**Europäisches Patentamt**

**European Patent Office**

**Office européen des brevets**

(11) Veröffentlichungsnummer: **0 290 981 B1**

(12) **EUROPÄISCHE PATENTSCHRIFT**

(45) Veröffentlichungstag der Patentschrift: **22.07.92**

(51) Int. Cl.⁵: **A61K 7/32, A61L 9/01**

(21) Anmeldenummer: **88107354.8**

(22) Anmeldetag: **07.05.88**

Die Akte enthält technische Angaben, die nach dem Eingang der Anmeldung eingereicht wurden und die nicht in dieser Patentschrift enthalten sind.

(54) **Deodorant.**

(30) Priorität: **14.05.87 DE 3716129**

(43) Veröffentlichungstag der Anmeldung:
**17.11.88 Patentblatt 88/46**

(45) Bekanntmachung des Hinweises auf die Patenterteilung:
**22.07.92 Patentblatt 92/30**

(84) Benannte Vertragsstaaten:
**AT BE CH DE ES FR GB GR IT LI LU NL SE**

(56) Entgegenhaltungen:
**BE-A- 903 646**
**DE-A- 2 611 895**
**FR-A- 1 533 371**
**GB-A- 1 008 281**

**CHEMICAL ABSTRACTS, Band 76, 1972, Columbus, OH (US); M.C.BARUZZI et al., Seite 321, Nr. 158212z**

**G. GARNIER et al., "Ressources médicinales de la flore française", Band I, 1961, Vigot Frères Editeurs, Paris (FR); Seiten 373-375**

**H. JANISTYN, "Handbuch der Kosmetika und Riechstoffe", I. Band: "Die kosmetischen Grundstoffe", Auflage 3, 1978, Dr. Alfred Hüthig Verlag, Heidelberg (DE); Seiten 189-191**

(73) Patentinhaber: **BGB Gesellschaft Wolfgang Krohn / Firma Krohn & Co. Verwaltungsgesellschaft (GmbH & Co.)**
**An der Alster 85**
**W-2000 Hamburg 1(DE)**

(72) Erfinder: **Meyer, Holger**
**Elfenbeinweg 15**
**W-2000 Hamburg(DE)**

(74) Vertreter: **UEXKÜLL & STOLBERG Patentanwälte**
**Beselerstrasse 4**
**W-2000 Hamburg 52(DE)**

## Beschreibung

Die Erfindung betrifft ein Deodorant, das sich insbesondere für die Verwendung in Zusammenhang mit Raumluftverbesserern und Körperdesodorantien eignet.

Es wurde überraschend gefunden, daß Kombinationen von Extrakten aus Coffea arabinensis und Rhabarber sowie zusätzlich gegebenenfalls Thea sinensis überlegene desodorierende Eigenschaften zeigen.

Es ist bekannt, daß Präparate, die Kaffee-Extrakte oder pulverisierte grüne Kaffeebohnen enthalten, desodorierende Eigenschaften aufweisen (siehe z.B. FR-A-1 533 371, GB-A-1 008 281, DE-A-2 611 895 und BE-A-903 646).

In der DE-A 16 17 598 ist ein Schönheitspflegemittel zur Hautbehandlung auf Wasser- und/oder Fettbasis beschrieben, daß einen Gehalt an wäßrigem und/oder fettigem Teextrakt aufweist. In dieser Druckschrift finden sich aber keine Angaben dahingehend, daß das beschriebene Schönheitspflegemittel oder die darin enthaltenen Teextrakte irgendeine desodorierende Wirkung aufweisen.

Gegenstand der Erfindung ist dementsprechend ein Deodorant, das dadurch gekennzeichnet ist, daß es einen Extrakt aus Coffea arabinensis und einen Extrakt aus Rhabarber enthält.

Die Herstellung der erfindungsgemäß zu verwendenden Extrakte erfolgt durch übliche Extraktion der erfindungsgemäß zu verwendenden Pflanzen bzw. Pflanzenteile. Bezüglich der geeigneten herkömmlichen Extraktionsverfahren wie der Mazeration, der Wirbelextraktion, der Gegenstromextraktion, der Perkolation, der Reperkolation, der Evakolation (Extraktion unter vermindertem Druck) und der Diakolation, die dem Fachmann geläufig und im Prinzip alle anwendbar sind, sei der Einfachheit halber beispielsweise auf Hagers Handbuch der Pharmazeutischen Praxis, 4. Aufl., Bd. 7, Springer-Verlag, Berlin-Heidelberg-New York 1971 verwiesen. Als Lösungsmittel für die Durchführung der Extraktion können organische Lösungsmittel, Wasser (vorzugsweise heißes Wasser mit einer Temperatur von mehr als 80°C und insbesondere mehr als 95°C) oder Gemische aus organischen Lösungsmitteln und Wasser, insbesondere niedermolekulare Alkohole mit mehr oder weniger hohen Wassergehalten (üblicherweise erfolgt die Extraktion mit derartigen Gemischen bei etwa Zimmertemperatur), verwendet werden. Als für die erfindungsgemäßen Zwecke besonders geeignet haben sich Ethanol-Wasser-Gemische (60 : 40) erwiesen.

Für die Extraktion von Coffea arabinensis wird Rohkaffe (nicht geröstete Kaffeebohnen) zerkleinert, und zwar zu einer Teilchengröße, die in etwa grobem Filterkaffee entspricht. Für die Extraktion von Thea sinensis werden in entsprechender Weise getrocknete oder auch nicht getrocknete Teeblätter mit einer Mühle oder einem Häcksler zu grobem Mehl zerkleinert. In gleicher Weise wird auch Rhabarber für die Extraktion vorbereitet. Bei Rhabarber kann jeweils die ganze Pflanze für die Extraktion herangezogen werden.

Die Extraktion kann bis zu jedem beliebigen Extraktionsgrad (soweit wirtschaftlich vertretbar) erfolgen, wird aber gewöhnlich bis zur Erschöpfung durchgeführt, wobei ein Extrakt erhalten wird, der in der Regel einen Feststoffgehalt von etwa 3-5 Gew.% aufweist. Für die Weiterverarbeitung des so erhaltenen Extraktes ist es meist vorteilhaft, durch Rückverdünnung mit Wasser einen niedrigeren Feststoffgehalt von etwa 1,5 Gew.% einzustellen. Der so erhaltene Dünnextrakt wird klar filtriert und unter Vakuum schonend aufkonzentriert, indem das im bevorzugten Extraktionsmittel (Ethanol-Wasser-Gemisch) enthaltene Ethanol abdestilliert wird, so daß dies für weitere Extraktionen eingesetzt werden kann. Anschließend wird dem aufkonzentrierten, von Ethanol befreiten Extrakt soviel Propylenglykol zugesetzt, daß der Feststoffgehalt wieder bei etwa 1,5 Gew.% liegt und das Lösungsmittel vorzugsweise aus etwa 40 % Propylenglykol und 60 % Wasser besteht. Dieser Extrakt dient dann als Komponente für Raumluftverbesserer- oder Körperdeodorantrezepturen oder andere Desodorierungsmittelrezepturen.

Die beschriebene Herstellung des in den Desodorierungsmittelrezepturen einzusetzenden Extrakts kann selbstverständlich vom Fachmann in Abhängigkeit vom vorgesehenen Anwendungszweck in verschiedenster Weise variiert werden. Dies stellt aber für den Fachmann keine Schwierigkeiten dar, so daß sich Erläuterungen hierzu erübrigen.

Die erfindungsgemäß verwendeten Extrakte enthalten Schleimstoffe, Gerbsäuren und Pflanzensäuren sowie die jeweils arttypischen Inhaltsstoffe der verwendeten Pflanzenarten. Ohne sich an eine Theorie binden zu wollen, wird angenommen, daß die Bindung der olfaktorischen Komponenten zunächst als Absorption im üblicherweise stark hygroskopischen Milieu der als solcher herkömmlichen Basiszubereitung des Deodorants erfolgt. Anschließend erfolgt wahrscheinlich eine clathratartige als auch chemische Bindung an die Inhaltsstoffe wie Stärke und Gerbsäure. Bei der Anwendung als Körperdeodorant werden dann die zusätzlichen Effekte der anderen Pflanzeninhaltsstoffe wirksam. Es handelt sich hierbei wahrscheinlich im wesentlichen um die Wirkung der Phytosterine, die zum einen sekretionshemmend und zum anderen antioxidativ wirken. Hierdurch wird die Menge der abgegebenen Stinkstoffe deutlich reduziert (Wirkung auf apokrine Drüsen) sowie auch deren oxidative Zersetzung vermindert.

Von besonderer Bedeutung dürfte weiterhin die aus dem Rohkaffee extrahierte Chlorogensäure sein, die bakteriostatisch wirkt und damit die bakterielle Zersetzung der olfaktorischen Komponente als Hauptursache des Körpergeruches verhindert. Die hohe Substantivität, d. h. das hohe Aufziehvermögen, dieser natürlichen antibakteriellen Substanz gewährleistet eine lang anhaltende Desodorierungswirkung, die sich mit der Reinsubstanz zwar nachweisen läßt, in der erfindungsgemäßen Gesamtzubereitung in Gegenwart der anderen Inhaltsstoffe jedoch eine erheblich bessere Wirkung entfaltet.

Es hat sich erfindungsgemäß üherraschend gezeigt, daß Deodorantien, die die gemäß Patentanspruch 1 verwendbaren Extrakte enthalten, einen deutlichen Synergismus der desodorierenden Wirkung zeigen, d. h. die Wirkung übersteigt bei weitem die Wirkung jeder Einzelkomponente.

Außerdem kann ein Extrakt aus Thea sinensis zugesetzt werden (siehe oben).

Die erfindungsgemäß zu verwendenden Extrakte dienen als Bestandteile in herkömmlichen Deodorantrezepturen, wobei sich die verwendete Menge nach dem Anwendungszweck und der gewünschten Effektivität richtet. Im allgemeinen beträgt die Konzentration der erfindungsmäß zu verwendenden Extrakte 0,5 bis 20 Gew.% und vorzugsweise 1 bis 10 Gew.%. Herkömmliche Deodorantrezepturen sind dem Fachmann bekannt. Es sei hierzu beispielsweise auf Karlheinz Schrader, Grundlagen und Rezepturen der Kosmetika, 1979, Dr. A. Huethig Verlag, Heidelberg und G. A. Nowak, Die kosmetischen Präparate, 2. Auflage 1975, Verlag für chem. Industrie H. Ziolkowsky, Augsburg verwiesen.

Beispiel 1

Rezeptur für einen Raumluftverbesserer:

| 5 % | Extrakt |
|---|---|
| 3 % | Triethylenglykol |
| 3 % | 1,2-Propylenglykol |
| 5 % | Isopropanol |
| 4 % | Wasser |
| 80 % | Treibgas |

Der Extrakt besitzt einen Feststoffgehalt von etwa 1,5 Gew.% und das Lösungsmittelgemisch besteht aus 40 Gew.% Propylenglykol und 60 Gew.% Wasser. Als Treibgas können die üblichen Treibgase wie Frigene, Kohlensäure usw. eingesetzt werden.

Beispiel 2

Rezeptur für einen Raumluftverbesserer zum Versprühen:

| 2-10 % | Extrakt |
|---|---|
| 3 % | Triethylenglykol |
| 3 % | Dipropylenglykol |
| Rest auf 100 % | Wasser |

Beispiel 3

Rezeptur für Roll-on Deodorant:

| 3 % | Polyglykol 400 |
|---|---|
| 2 % | Extrakt |
| 30 % | Ethanol |
| 15 % | Wasser |
| 50 % | Verdickungsmittel auf Methylcellulosebasis |

Beispiel 4

Rezeptur für einen Deodorant-Spray:

| 1 % | Extrakt |
|---|---|
| 4 % | Wasser |
| 25 % | Ethanol |
| 70 % | Treibgas |

Hinsichtlich der in den Beispielen 3 bis 5 eingesetzten Extrakte und Treibgase sei auf die Erläuterungen in Beispiel 1 verwiesen.

**Patentansprüche**

**Patentansprüche für folgende Vertragsstaaten : AT, BE, CH, DE, FR, GB, GR, IT, LI, LU, NL, SE**

1. Deodorant, dadurch gekennzeichnet, daß es einen Extrakt aus Coffea arabinensis und einen Extrakt aus Rhabarber enthält.

2. Deodorant nach Anspruch 1, das zusätzlich einen Extrakt aus Thea sinensis enthält.

3. Verwendung des Deodorants nach Anspruch 1 oder 2 als Raumluftverbesserer oder Körperdeodorant.

**Patentansprüche für folgenden Vertragsstaat : ES**

1. Verfahren zur Herstellung eines Deodorants auf Basis herkömmlicher Deodorantrezepturen, **dadurch gekennzeichnet,** daß man
   1. einen Extrakt aus Coffea arabinensis herstellt, indem man Rohkaffee zerkleinert und dann mit einem Lösungsmittel extrahiert,
   2. einen Extrakt aus Rhabarber herstellt, indem man Rhabarber zerkleinert und dann mit einem Lösungsmittel extrahiert, und
   3. die in den Stufen 1 und 2 gewonnenen Extrakte in herkömmliche Deodorantrezepturen einarbeitet.

2. Verfahren nach Anspruch 1, bei dem man zusätzlich einen Extrakt aus Thea sinensis herstellt, indem man Teeblätter zerkleinert und dann mit einem Lösungsmittel extrahiert, und den erhaltenen Extrakt ebenfalls in die Deodorantrezeptur einarbeitet.

3. Verfahren nach Anspruch 1 oder 2, bei dem man Extrakte mit einem Feststoffgehalt von 1,5 Gew.% herstellt und diese Extrakte in einer Menge von 0,5 bis 20 Gew.% in die Deodorantrezeptur einarbeitet.

4. Verwendung des nach dem Verfahren gemäß einem der Ansprüche 1 bis 3 hergestellten Deodorants als Raumluftverbesserer oder Körperdeodorant.

**Claims**

**Claims for the following Contracting States : AT, BE, CH, DE, FR, GB, GR, IT, LI, LU, NL, SE**

1. Deodorant, thereby chracterised, that it contains an extract of Coffea arabinensis and an extract of rhubarb.

2. Deodorant according to claim 1, which additionally contains an extract of Thea sinensis.

3. Use of the deodorant according to claim 1 or 2 as a space air freshener or body deodorant.

**Claims for the following Contracting State : ES**

1. Process for the production of a deodorant on the basis of conventional deodorant formulations, **thereby characterised,** that one
   1. prepares an extract of Coffea arabinensis, by comminuting raw coffee and then extracting with a

4

solvent,

2. prepares an extract of rhubarb, by comminuting rhubarb and then extracting with a solvent, and

3. incorporates the extracts obtained in steps 1 and 2 in conventional deodorant formulations.

**2.** Process according to claim 1, in which one additionally prepares an extract of Thea sinensis, by comminuting tea leaves and then extracting with a solvent, and likewise incorporates the resultant extract in the deodorant formulation.

**3.** Process according to claim 1 or 2, in which one prepares extracts with a solids content of 1.5 wt. % and incorporates these extracts in an amount of 0.5 to 20 wt. % in the deodorant formulation.

**4.** Use of the deodorant prepared by the process according to any of claims 1 to 3 as space air freshener or body deodorant.

## Revendications

**Revendications pour les Etats contractants suivants : AT, BE, CH, DE, FR, GB, GR, IT, LI, LU, NL, SE**

**1.** Désodorisant, caractérisé en ce qu'il contient un extrait de Coffea arabinensis et un extrait de rhubarbe.

**2.** Désodorisant selon la revendication 1, contenant en outre un extrait de Thea sinensis.

**3.** Utilisation du désodorisant selon la revendication 1 en tant que désodorisant d'atmosphère et déodorant corporel.

## Revendications pour l'Etat contractant suivant : ES

**1.** Procédé de préparation d'un désodorisant sur la base de formulations de désodorisant classiques, caractérisé en ce que l'on :

1. prépare un extrait de Coffea arabinensis, en broyant du café brut que l'on extrait ensuite à l'aide d'un solvant,

2. prépare un extrait de rhubarbe, en broyant de la rhubarbe que l'on extrait ensuite à l'aide d'un solvant, et

3. incorpore les extraits obtenus aux étapes 1 et 2 dans des formulations de désodorisant classiques.

**2.** Procédé selon la revendication 1, dans lequel on prépare en outre un extrait de Thea sinensis, en broyant des feuilles de thé que l'on extrait ensuite au moyen d'un solvant, et incorpore également l'extrait obtenu dans une formulation de désodorisant.

**3.** Procédé selon la revendication 1 ou la revendication 2, dans lequel on prépare des extraits ayant une teneur en matières solides de 1,5%, en poids et on incorpore ces extraits en une quantité de 0,5 à 20%, en poids dans la formulation de désodorisant.

**4.** Utilisation du désodorisant préparé selon l'une des revendications 1 à 3 en tant que désodorisant d'atmosphère ou déodorant corporel.